(19) Europäisches Patentamt
European Patent Office
Office européen des brevets

(11) EP 2 246 386 A1

(12) **EUROPEAN PATENT APPLICATION**

(43) Date of publication:
**03.11.2010 Bulletin 2010/44**

(21) Application number: **09158848.3**

(22) Date of filing: **27.04.2009**

(51) Int Cl.:
***C08J 7/12*** *(2006.01)* ***A61L 27/34*** *(2006.01)*
***A61F 2/16*** *(2006.01)*

(84) Designated Contracting States:
**AT BE BG CH CY CZ DE DK EE ES FI FR GB GR HR HU IE IS IT LI LT LU LV MC MK MT NL NO PL PT RO SE SI SK TR**

(71) Applicants:
- **Université de Liège**
  **4031 Angleur (BE)**
- **Physiol S.A.**
  **B-4031 Angleur (BE)**

(72) Inventors:
- **Bozukova, Dimitriya**
  **B-4031, Liege (BE)**
- **Jerome, Christine**
  **BE 4000, LIEGE (BE)**
- **Pagnoulle, Christophe**
  **4031, ANGLEUR (BE)**

(54) **Flourinated material**

(57) This invention relates to a method for modifying the surface of a material for use with a biological sample or tissue comprising the steps of providing a material having a surface which comprises reactive-functional groups; providing fluorinated molecules having reactive-functional groups complimentary to those on the material surface; using wet chemistry to attach the fluorinated molecules to the surface of the material by reacting the reactive-functional groups of the implant with the complementary reactive-functional groups of the fluorinated molecules. The invention also relates to materials modified by said method and to implants made using said materials.

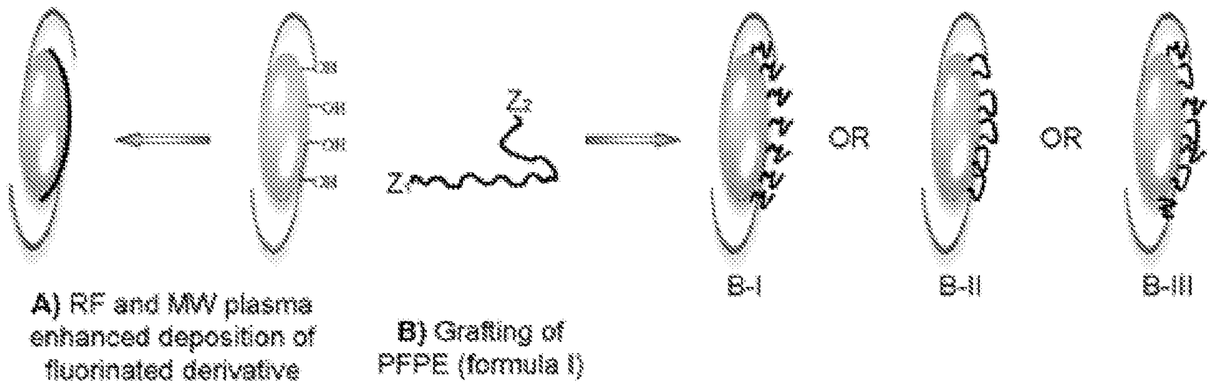


Figure 1

EP 2 246 386 A1

## Description

The present invention relates to the field of materials for use in biological systems, in particular to materials for use in medical implants, and to methods for the surface modification of such materials, to materials with a surface modification, and to the use of materials with a surface modification.

Implants are often used in medical applications to replace damaged or lost tissue. Such implants are often made from polymers which have good biocompatibility and have the desired mechanical properties. The interaction of the surface of an implant with the surrounding cells or fluid is a particularly important consideration for implant design, material and preparation. Inappropriate cell growth and attachment on the surface of an implant may lead to failure of the implant.

Common areas of use for medical implants, and in particular, implants made from polymers, are orthopaedic, cardiac and eye applications. For example, as a steadily increasing number of people suffer from cataracts which require surgical intervention in order to extract an opacified cataractous eye lens, the need to replace the lens with a synthetic intraocular lens (IOL) implant grows. Although this surgery is usually successful, posterior capsular opacification may occur as a result of the adhesion and growth of lens epithelial cells (LECs) on the posterior side of the intraocular lens implant. Whilst Nd:YAG laser capsulotomy may be used to treat posterior capsular opacification, it may result in severe complications. Thus, prevention of posterior capsular opacification is a more desirable strategy. One approach to prevent posterior capsular opacification may be to modify the surface of the intraocular lens implant.

The biocompatibility of implant surfaces is well-known to depend on the wettability and topography of the surface. Guelcher et al. (2003. An introduction to biomaterials. Taylor Francis Group) reported that the ability of biomaterials to repel cells depends on their wettability. They established that very hydrophobic and very hydrophilic materials exhibit effective cell repellency, in contrast to surfaces of intermediate wettability. This observation is consistent with the high rate of cell adhesion onto the surface of polymers usually used in the production of intraocular lens implants, such as poly(methyl methacrylate) [poly(MMA)], poly(hydroxyethyl methacrylate) [poly(HEMA)], poly(HEMA-*co*-MMA), silicone, etc. It appears that posterior capsular opacification occurs at a rate that depends on the chemical composition of the constitutive polymer.

In a series of patents, i.e. US 2002/0051448 Al, US 2005/6943204 B2 and EP 2007/1860141 Al, Wang et al. describe a two-step process for the grafting of heparin on to the surface of intraocular lens implants, preferably made of silicone, to create a hydrophilic cell repellent surface. Initially a layer of interpenetrating networks is formed on the implant surface, by swelling the top layer with a functional monomer with consequent polymerization. The penetrating monomer functionalities are then reacted with heparin in order to graft the heparin on to the surface of the intraocular lens implant.

Alternatively, phospholipids containing the monomer 2-methacryloyloxyethyl phosphoryl-choline (MPC), have been used as surface modification agents with silicone implants. These agents demonstrate properties, such as, low protein adsorption, enhanced cell repulsion, and inertness in contact with biological systems (Yao, K. et al. 2006, J Biomed. Mater. Res. 78A, 684-692; Willis, S.L. et al. 2001. Biomaterials 2001, 22, 3261-3272; Hirota, K. et al. 2005. FEMS Microbiol. Lett. 248, 37-45; Moro, T. et al. 2004. Nat. Mater., 3, 829-836).

The formation of a hydrophobic perfluorinated coating on the surface of an intraocular lens implant is another approach suggested to impart cell repellent properties to the implant. For example, US patent application 2002/0028330 Al describes the formation of semi-fluorinated alkyl hydrocarbon polymers on the surface of intraocular lens implants, by dip-or spin-coating the implant with butyl acetate graft-polymer solution, followed by exposure to plasma, during which grafting is expected.

EP 1997/0487418 Bl describes the formation of a perfluorinated coating on the surface of an intraocular lens implant by radio-frequency (RF) plasma enhanced deposition of non-polymerizable fluorinated precursor, such as $CF_4$ and $SF_6$.

WO 1988/08287 and US 1992/5091204 describe a further method to create a cell repellent, dense, impermeable, rigid, cross-linked film of radio-frequency plasma polymerized perfluoropropane on the surface of a poly(MMA) intraocular lens implant.

According to a first aspect of the present invention, there is provided a method for modifying the surface of a material for use with a biological sample or tissue comprising the steps:

- providing a material having a surface which comprises reactive-functional groups;
- providing fluorinated molecules having reactive-functional groups complementary to those on the material surface;
- using wet chemistry to attach the fluorinated molecules to the surface of the material by reacting the reactive-functional groups of the material with the complementary reactive-functional groups of the fluorinated molecules.

"Wet chemistry" means a reaction performed in the liquid state. Preferably, wherein one liquid reactant or one solution of the reactant is a solvent.

Surprisingly, by using wet chemistry to attach fluorinated molecules to a material surface, a surface with much improved properties is obtained, for example, the surface preferably displays one or more of the following properties: improved hydrophobicity; improved cell repellency; improved ability to resist deformation; improved optical properties; and improved ability to withstand sterilisation. Particularly, the properties are improved when compared to plasma treated surfaces.

According to another aspect of the present invention, there is provided a method for modifying the surface of a material for use with a biological sample or tissue comprising the steps:

- providing a material having a surface which comprises reactive-functional groups;
- providing fluorinated molecules having reactive-functional groups complementary to those on the material surface;
- attaching the fluorinated molecules to the surface of the material by reacting the reactive-functional groups on the material with the complementary reactive-functional groups of the fluorinated molecules, to form a brush of fluorinated molecules on the surface of the material.

The material modified by any method of the invention may be used to form an implant, preferably a medical implant, or it may form a surface for culturing cells or a tissue, or it may form the surface of a medical or surgical device or instrument. Preferably the material forms all or part of an implant. The material may be fluorinated before or after it have been formed into an implant or other product.

The attachment of the fluorinated molecules to the surface of the material is preferably not achieved by plasma deposition.

Preferably the surface of the material is modified by any method of the invention to form a brush of flourinated polymer on the material surface. In the brush of polymer the fluorinated molecules are preferably not cross linked to each other, preferably at least 50%, 60%, 70%, 80%, 85%, 90%, 95% or more of the fluorinated molecules are not cross linked to one or more other fluorinated molecules. The fluorinated molecules may be tethered to the surface by one or two anchoring sites. If anchored at two sites the fluorinated molecules may form a loop on the material surface.

Preferably plasma deposition cannot form a brush of fluorinated molecules on a material surface, even if liquid precursors are used. Instead plasma deposition of fluorocarbons produces a surface on which the fluorinated molecules are crosslinked to both the material surface and each other.

The fluorinated molecules may be provided or attached in a liquid phase. Preferably, the fluorinated molecules are not provided or attached in a gaseous or vapour phase. Preferably the reacting step is conducted in solution.

The attaching of the fluorinated molecules to the material surface may be achieved by covalently-linking/bonding the fluorinated molecules to the material, in particular, to the surface of the material, preferably to the surface of an implant.

The reactive-functional groups on the material surface may include one or more initiators of polymerization, including controlled/living polymerization, such as CRP, anionic or cationic polymerisation, the reactive-functional groups may include one or more of -NH2, -COOH, -OH, -COC1, -NCO, -COBr, -CN, or $-N_3$. The reactive functional group on the fluorinated molecule may include one or more of $CH_2=CHCOO-$, $CH_2=C(CH_3)COO-$, oxirane, alcyne, anhydride, -CHO, $-NH_2$, -COOH, -OH, -COC1, -NCO, -COBr, -CN, or $-N_3$.

Preferably, once fluorinated, the surface of the material is hydrophobic.

Preferably, once fluorinated, the surface of the material repels cell binding.

Preferably, if the material is used in an implant the surface of the implant is hydrophobic and repels cell binding.

Preferably a hydrophobic surface has a contact angle of water in air of above 70° (Guelcher et al. 2003 An introduction to biomaterials Taylor Francis Group.)

Preferably cell binding is repelled if the amount of cells which bind to a treated/modified surface is less than that which would bind to an untreated surface, preferably the method of the invention results in an at least an about 10%, 20%, 30%, 40%, 50%, 60%, 70% 80%, 90% or more reduction in cell binding when compared to the same surface untreated. The degree of repulsion will depend on the surface used and the type of cells.

The method of the invention has the benefit that it can provide a soft coating of fluorinated molecules, which can accommodate mechanical stresses, for example, an IOL implant made using a material fluorinated according to the method of the invention is sufficiently flexible to withstand mechanical stresses caused by the swelling of the core of the IOL implant. This is in contrast to plasma deposited fluorinated coatings/modifications which form rigid cross-linked films which if used to coat an IOL implant cannot stand the mechanical stresses of use, and which exhibit cracks upon swelling of the implant core.

A soft coating may be defined as a coating with no, or no significant, cross linking between the fluorinated molecules. The absence of any, or any significant cross linking implies more chain mobility and thus coating flexibility.

Preferably, surfaces modified by the method of the invention using wet chemical reactions are stable over time and/or are uniform across the surface, this is in contrast to surfaces modified by plasma deposition. Surfaces modified by plasma deposition tend to have an unpredictable stability and to lack uniformity.

Preferably, the fluorinated molecules are perfluorinated.

A "fluorinated molecule" may be defined as a compound or molecule in which at least one fluorine has been incorporated. A "perfluorinated molecule" may be defined as a compound or molecule, where many or most hydrogen atoms have been substituted by fluorine atoms.

Preferably the fluorinated molecules are hydrophobic.

The fluorinated molecules may be oligomers or polymers. The fluorinated molecules may be homomeric or they may be copolymers of two or more different monomers.

Preferably the fluorinated molecules are hydrophobic oligomers or polymers, such as perfluorinated polyethers (PFPE). The fluorinated molecules may be end-capped, at one or more chain-ends, by reactive-functional groups able to react with reactive-functional groups available on the material surface.

The fluorinated polymer or oligomer may be a fluorinated, or perfluorinated, polyether. Preferably the fluorinated polymer or oligomer is of the Formula I:

Formula I: $$Z_1 - (PFPE_1)_m - (PFPE_2)_n - Z_2$$

$PFPE_1$ is 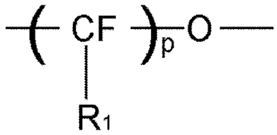

$PFPE_2$ is 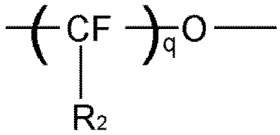

$Z_1$ is a reactive functionality, which might be, but is not limited to, - CH2=CHCOO-, CH2=C(CH3)COO-, oxirane, alcyne, anhydride, -CHO, - NH2, -COOH, -OH, -COCl, -NCO, -COBr, -CN, -N3,...

$Z_2$ might be either-H; -F; $-CH_3$; $-CF_3$; $-CF_2H$; $-CFH_2$,

or a reactive functionality, which might be, but is not limited to, CH2=CHCOO-, CH2=C(CH3)COO-, oxirane, alcyne, anhydride, -CHO, - NH2, -COOH, -OH, -COCl, -NCO, -COBr, -CN, -N3,...

$R_1$ is H, F, $CH_3$, $CF_3$, $CHF_2$,$CH_2F$;

$R_1$ is H, F, $CH_3$, $CF_3$, $CHF_2$,$CH_2F$;

**m** and **n** might have the same values, or different; and are equal or greater than 1; or not more than one might be equal to 0, the other one being equal or greater than 1;

**p** and **q** might have the same values, or different; and are between 1 and 5.

$PFPE_1$ and $PFPE_2$ in Formula I may be the same or different, and may be randomly distributed or distributed as discrete blocks throughout the chain.

A perfluorinated polyether for use in the invention may comprise a single, or repeated, unit of the formula $-(CF_2-CF_2-O)-$.

Perfluorinated polyethers are particularly advantageous because they are highly hydrophobic and are less crystalline than, for example, perfluoroalkyl chains $-(CF_2-CF_2)n-$. The presence of the oxygen as an ether does not significantly influence the hydrophobicity of the molecules, but it does reduce their crystallinity. Thus a coating/modification formed using such polyether molecules may be softer and more able to accommodate mechanical stresses, for example, during the swelling of a material, in particular during the swelling of the core of a material. Perfluoroether oligomers or polymers may also offer further advantageous surface properties, including low friction, low surface tension, high oxygen permeability, good biocompatibility and good thermal and chemical stability. Moreover, these types of polymers or oligomers resist biodegradation, because of a lack of oxidizable moieties and abstractable hydrogen atoms.

Other fluorinated molecules which may be used in the method of the invention include perfluoro(meth)acrylate, fluoropolyethylene, perfluorostyrene, perfluoropolyurethanes and $\alpha,\omega$-diisocyanate poly(perfluoro ethylene oxide-co-perfluoromethylene oxide)

The reactive-functional groups on the implant surface may be selected from any of the group comprising -NCO; -COOH; - COCl; -COBr; -CHO; -OH; -CN, $-N_3$ and $-NH_2$, or combinations thereof.

The complementary reactive-functional groups of the fluorinated molecules may be selected from any of the group comprising $CH_2=CHCOO-$, $CH_2=C(CH_3)COO-$, oxirane, alcyne, anhydride, -CHO, $-NH_2$, -COOH, -OH, -COCl,

-NCO, -COBr, -CN, or -$N_3$ or combinations thereof.

The reactive-functional groups on the surface of the material may be inherent in the material, or incorporated into the material during its manufacture, or applied to the material post manufacture.

In an alternative embodiment the reactive-functional groups may be provided by plasma modification of a material surface prior to the attachment of the fluorinated molecules. The plasma modification may be radio-frequency plasma modification or microwave plasma modification. The material, and accordingly any implant comprising the material, may be rigid or non-rigid.

The material, and accordingly any implant comprising the material, may be foldable/compressible.

The material may be used in an implant, the implant may be a medical implant or device for implantation into the human or animal body. The implant may be an intraocular lens implant or a breast implant. Alternatively, the material may be used in hydrogel wound dressings, in surgical sealants, in drug delivery reservoirs, in skeletal implants/scaffolds, in vertebral expanders, in tubings for catheters, stents, drains and/or dialyzers, to name but a few examples.

The material may also be used for other articles in contact with biological samples, such as living tissues or living cells, for example, in petri dishes and cells culture vessels.

The material, and hence an implant comprising the material, may have a refractive index of about 1.40 or greater.

Preferably, the surface modification according to the invention does not substantially affect the transparency of the material. In particular, if the material is to be used in an intraocular lens implant, preferably the method of the invention does not affect transparency of the implant material.

The material may be selected from any of the group comprising silicone polymers; hydrocarbon polymers; hydrogels; acrylic polymers; polyesters; polyamides; polyurethanes; and silicone polymers with hydrophilic monomer units; or combinations thereof. Alternatively, the material may be glass, metal or any other suitable material.

The material may be hydrophilic prior to surface modification. Preferably if the material is to be used to form an IOL implant the material prior to surface modification according to the invention is hydrophilic.

The material may be a water-swelling hydrogel. A hydrogel may comprise a network of hydrophilic polymer chains that are water-insoluble because of cross-linking but able to swell in water. When hydrated, the hydrogel may be capable of having a water content of between about 10% and about 1000% by weight.

Preferably the material comprises a hydrogel material comprising HEMA or MMA, or a copolymer thereof, for example poly(HEMA-*co*-MMA).

In an embodiment where the material is to be used in an intraocular lens implant, poly(HEMA-*co*-MMA) is an advantageous polymeric material to use due to its equilibrium water content from about 5 to about 50 w%, and a high refractive index from about 1.35 to about 1.80, which is greater than that of the aqueous humour of the eye, which is 1.336. A high refractive index is a desirable feature in the production of an intraocular lens implant to impart high optical power with a minimum of optic thickness. Using a material with a high refractive index may allow visual acuity deficiencies to be corrected using a thinner intraocular lens implant.

Poly(HEMA-*co*-MMA) is also an advantageous material in the production of intraocular lens implants, and indeed any implant, due to its mechanical strength. Implants made from poly(HEMA-*co*-MMA) are able to withstand considerable physical manipulation. Poly(HEMA-*co*-MMA) also has desirable shape-recovery properties for use in intraocular lens implants. Intraocular lens implants manufactured from a material possessing desirable shape-recovery properties, such as poly(HEMA-*co*-MMA), unfold in a more controllable manner when implanted in an eye, rather than explosively, to form its predetermined shape. Explosive unfolding of intraocular lens implants is undesirable due to potential damage to delicate tissues within the eye. Poly(HEMA-*co*-MMA) in an IOL implant also provides dimensional-stability in the eye, which is likewise advantageous.

Dimensional stability should be understood as the ability of a material to memorize its form after extreme or repeated deformation (eye focussing sometimes leads to deformation of the IOL).

The method may further include the step of sterilisation of the material, preferably after the attaching of the fluorinated molecules, such as prior to use.

Material treated according to the method of the invention may be sterilised by any suitable sterlisation method, for example, by UV or gamma rays, by ethylene oxide treatment, by steam treatment (autoclaving, eg. 120°C and 1.5 bar for 21 min (standard conditions for IOLs can be found in Dumaine, L. Thesis. Ecole Centrale de Lyon. 2004, 79-85; Oehr, C. Nuclear Instrum. Methods Phys. Res. 2003, 208,40-47). In particular, a material treated by the method of the invention is preferably compatible with water vapour sterilisation.

An advantage of the modified material provided by the method of the invention is that it can be subject to sterilisation without significantly affecting the benefits provided by the surface modification of the present invention.

According to another aspect of the present invention, there is provided a material having a surface modification provided by any method of the present invention. Preferably the material is used in an implant, such that the implant has a surface modification provided by any method of the invention.

According to another aspect of the present invention, there is provided a material, such as that used in an implant, comprising fluorinated polyether attached to a surface thereof.

According to another aspect of the present invention, there is provided the use of a material having a surface modification provided by any method of the present invention, the material may be used in an implant.

According to another aspect of the present invention, there is provided fluorinated polyether for use in the modification of the surface of a material, such as that used in an implant.

According to another aspect of the present invention, there is provided fluorinated polyether for use on the surface of material for the prevention of cell growth and/or adherence on the material.

The fluorinated polyether may be provided for use on the surface of an intraocular lens implant for the prevention of posterior capsular opacification.

The fluorinated polyether may be a compound according to Formula I.

According to another aspect of the present invention, there is provided a method of reducing or preventing posterior capsular opacification comprising modifying a surface of an IOL implant by applying the method of the invention to the implant.

It will be appreciated that, where appropriate, all optional or preferable features applicable to one aspect of the invention can be used in any combination, and in any number. Moreover, they can also be used with any of the other aspects of the invention in any combination and in any number. This includes, but is not limited to, the dependent claims from any claim being used as dependent claims for any other claim in the claims of this application.

Embodiments and examples of the present invention will now be described herein, by way of example only, with reference to the following figures.

**Figure 1** - illustrates a schematic representation of the formation of an (A) plasma or a (B) chemical fluorinated coating/modification on the surface of poly(HEMA-co-MMA) hydrogel. PFPE coatings might consist of (B-I) one chain-end; (B-II) both chain-ends or (B-III) one and both chain-ends grafted PFPE molecules, where $Z_1$ and $Z_2$ are as in Formula I;

**Figure 2** - illustrates the Attenuated Total Reflectance Fourier Transform Infrared (ATR-IR) spectra of dry (A) unmodified and (B) PFDI chemical grafted hydrogel after sterilization;

**Figure 3** - shows a visualization of a water droplet on the dried surface of an unmodified and a PFDI chemical grafted hydrogel after sterilization;

**Figure 4** - shows AFM images of the surface of hydrated polished lenses modified by plasma and chemical grafting. Sample (A) unmodified polished; (A') unmodified non-polished; (B) PFDI-grafted; (C to F) RF fluorinated plasma, polished; (G, H, J) MW fluorinated plasma, polished; (L) MW fluorinated plasma 9 cm, non-polished; (O to R) MW fluorinated plasma II cm, non-polished; ( T, U and V) argon plasma for 30, 60 and 120 sec, respectively. For more details see Table 1.

**Figure 5** - illustrates the RMS roughness of hydrated lenses treated by (A) RF plasma and (B) MW plasma (for 60 sec)

**Figure 6** - illustrates the RMS roughness of hydrated lenses treated by MW argon- and fluorinated- plasmas

**Figure 7** - illustrates the RMS roughness of polished hydrated lenses before and after PFDI chemical grafting

**Figure 8** - illustrates percentage of the surface occupied by cells for lens implants treated by (A) RF plasma, (B) MW fluorinated- and argon- plasma, for different times; (C) chemical grafting of PFDI; and

**Figure 9** - illustrates the UV-Vis transmission of hydrated polished lens implant modified by (A) fluorinated MW plasma (9 cm apart from the plasma source); (B) fluorinated RF plasma and (C) chemical grafting of PFDI.

## EXPERIMENTS

The following examples apply to intraocular lens implants; however, the skilled person will appreciate that they can be applied to any appropriate material.

With reference to Figure 1, two strategies were compared for the surface fluorination of the poly(HEMA-*co*-MMA) copolymer, 1) plasma enhanced deposition of perfluoroalkanes and 2) chemical grafting of $\alpha,\omega$-diisocyanate poly(perfluoro ethylene oxide-co-perfluoromethylene oxide) oligomers (PFDI; Mn = 3000 g/mol). The results demonstrate the advantage of chemical grafting.

It will be understood by a skilled person that "chemical grafting" is intended to refer to a wet-chemical reaction, for example, it is not a plasma mediated reaction/deposition, or the like.

### *Selection of the experimental conditions for surface modification by wet chemistry*

The reactive precursor (PFDI) used in this work is a commercially available oligomer, end-capped at both ends by an isocyanate reactive-functional group, which is reactive towards the hydroxyl functional groups available at the surface of the intraocular lens implants (Evens, M.D.M. et al. 2001. Biomaterials, 22, 3319-3328). This reaction (80 °C, 48 h, 0.1 mol% dibutyltin dilaurate with respect to isocyanate) leads to the formation of a hydrolytically stable urethane bond (Klee, D. et al. 1999. Adv. Pol. Sci. 149, 1-57).

The PFDI molar mass of 3000 g/mol was thought to be suitable for coating the intraocular lens implants without adversely affecting their optical and mechanical performances. The low Tg of these oligomers meant that the coating should be flexible enough to be unaffected by any change in size of the lens implant, for example, due to swelling or deformation.

The grafting of some PFDI chains by only one of the two isocyanate end-groups may not be precluded, such that residual OCN-groups might be available at the lens surface. In this work, any residual OCN-groups groups were not removed. However, they could be removed if necessary, for example, by reaction with a low molecular weight alcohol or amine.

### *Selection of the experimental parameters for surface fluorination by plasma deposition*

Two types of plasma, i.e. microwave and radio-frequency plasma, were selected with the purpose of modulating the surface properties of the intraocular lens implants (Denes, F.S. & Manolache, S. 2004. Prog. Polym. Sci., 29, 815-885; Bogaerts, A. et al. 2002. Spectrochemica Acta Part B, 57, 609-658; and Chan. C.M. 1996. Surf Sci. Reports, 24, 1-54).

Two experimental parameters, namely the treatment duration and the distance between the implant surface and the plasma source, were systematically modified, as they are known to affect the surface topography and chemical composition. The treatment time was selected for each plasma method on the basis of data reported in the scientific literature. In particular, surface modifications by radio-frequency plasma are usually complete within 5 to 20 min (Senesi, G.S. et al. 2007. Surf Sci., 601, 1019-1025; US 5 091 204; Pizzoferrato, A. et al. 1995. Biomaterials, 16, 361-367; Vaswani, S. 2004. Academic Thesis. Georgia Institute of Technology; Garrison, M.D. et al. 1999. Thin Solid Films, 352, 13-21), which prompted the selection of 5, 10, 15 and 20 min in this work. Similarly, 30 sec was reported to be sufficient for microwave plasma depositing of fluorine, which remained constant upon extended treatment (Hochart, F. et al. 1999. Appl. Surf Sci. 42(1-4), 574-578). Therefore, the intraocular lens implants were modified by the microwave plasma for 30, 60, 90 and 120 sec, respectively. The distance between the surface to be modified and the plasma source was changed only for demonstration in the case of the microwave plasma method.

Perfluoropropane (PFP) was used for the radio-frequency plasma fluorination as Ratner et al. (US 5 091 204) did for the surface modification of poly(MMA) intraocular lens implants. Perfluoroethane (PFE) was used for the microwave plasma modification of the intraocular lens implants, which was found to be suitable for the plasma surface modification of biomaterials by Sterrett, T.L. et al (1992. J. Mater. Sci. Mater. Med., 3,402-407).

### *Materials*

Poly(HEMA-*co*-MMA) hydrogel intraocular lens implants (equilibrium water content of 26%) were provided by PhysIOL s.a. (Liège, Belgium), and disks (referred to herein as "the intraocular lens implants" or "lens implants") with the desired thickness of 1mm in the dry state were cut out with a diamond tool. Part of the disks/lens implants were further polished by a standard industrial procedure (such as described by Wrue et al, US Patent 6729939, 2003). Both the non-polished and polished disks/lenses were extracted with Milli-Q water in a Soxhlet extractor for 5 days, in order to eliminate any residual monomer and oligomer. They were then dried in a vacuum oven at 50 °C for 2 days and stored in a vial under ambient atmosphere. Just before use, they were dried again in vacuo (0.05mm Hg) at 80 °C overnight.

PFDI, dibutyltin dilaurate (DBTDL) and trifluorotoluene (TFT) were supplied by Aldrich and used as received. Water was purified by a MilliQ system from Millipore.

### *Microwave (MW) perfluoroethane (PFE) plasma*

MW plasma equipment (P400) was operated at 2.45 GHz and 150 W (800-1000 μs), with perfluoroethane (PFE, Halocarbon 116, 99.999%, Air products) as a fluorine precursor, at a flow rate of 50 sccm under 25 Pa. Both the treatment time (30 s, 60 s, 90 s and 120 s) and the distance of the substrate from the plasma source (9 cm and 11 cm) were varied.

***Radiofrequency (RF) perfluoropropane (PFP) plasma***

Intraocular lens implants were treated with CD300 PLC equipment operating at a frequency of 13.56 MHz and a pressure of 2.67 to 5.34 Pa, with a 300 W generator and perfluoropropane (PFP) as a fluorine precursor. Several treatment times were considered, i.e. 5, 10, 15 and 20 min, respectively. 20 min is a standard treatment time for the surface modification of materials used in biomedical applications.

***MW argon plasma***

As a control, non-polished lens implants were treated by MW argon plasma with ROTH&RAU AK 330 MW plasma equipment operating at 2.45 GHz. The other experimental conditions were 150 W (800-1000 μs), 25 Pa, 50 sccm argon flow, for 30 s, 60 s, 90 s and 120 s, respectively.

Whichever plasma method was used, the dry lens implants were stuck to a microscopic glass slide with double-sided tape. After plasma treatment, the lenses were detached from the slide and the unmodified face was carefully cleaned and marked. The treated lenses were then characterized whilst dry or after swelling by water.

***Surface grafting of PFDI by wet chemistry***

Polished intraocular lens implant samples were dried in a reaction flask in vacuo (0.05mm Hg) at 80 °C overnight. The flask was then purged with argon and a 50/50 (v/v) solution of PFDI in trifluorotoluene was added until the samples were completely immersed. The reaction was conducted at 80 °C for 48 h in the presence of a catalytic amount of dibutyltin dilaurate (0.1mol% with respect to isocyanate).

Unreacted PFDI was eliminated by repeated washing (three times) with trifluorotoluene at 50 °C (i.e. at the temperature at which PFDI is soluble in trifluorotoluene). The lens implants were then either dried in a vacuum oven at 50 °C for 2 days, or kept in Milli-Q water until characterization.

***Preparation of lens implants - hydration, drying and sterilization***

Dry lens implants, after surface modification, were used for measurement by Attenuated Total Reflectance Fourier Transform Infrared (ATR-IR), X-ray Photoelectron Spectroscopy (XPS) and contact angle measurements of water (by the 'water droplet' method), (*cfr infra*).

The surface-modified/treated lens implants were then swollen (hydrated) by Milli-Q water (at least for 48 h, at 25 °C) in order to investigate the stability of the coating/surface modification upon hydration/water-swelling of the implants. The hydrated/water-swollen lens implants were then used for contact angle measurements of air (by the 'captive bubble' technique), (*cfr infra*). The same specimens were then sterilized in Milli-Q water at 120 °C and 1.5 bar, for 21 min, in order to check the stability of the surface modification under sterilization conditions. The sterilized lens implants were again characterized by measurement of the contact angle of air ('captive bubble' method, *cfr infra*).

After drying in vacuo at 50°C for 48 h, the same lens implants were used for contact angle measurements of water by the 'water droplet' method, ATR-IR and XPS *(cfr infra).*

***Characterization of the coating***

***ATR-IR spectroscopy***

Formation and stability of the PFDI-coatings/modifications were confirmed by ATR-IR (Attenuated Total Reflectance Fourier Transform Infrared) spectroscopy (with a Nicolet spectrophotometer (Ge crystal) in the 800-1800 nm range) for pre-dried implants, before and after sterilization (Fig. 2). Indeed, the absorption at 3421 $cm^{-1}$ typical of the OH-groups of the original surface (Fig. 2A) dramatically decreased upon reaction with the isocyanate end-groups of PFDI. Formation of urethane groups (-NHCO-) was assessed by the appearance of an extra absorption at approximately 1550 $cm^{-1}$ (Fig. 2B). In parallel, the width of the absorption at 1233 $cm^{-1}$ increased (appearance of a shoulder), which is the signature of the C-F bonds of the PFDI-oligomers.

The ATR-IR spectra of the grafted samples did not change significantly upon sterilization (data not shown), which is evidence for the coating/modification stability.

***Contact angles data***

The contact angle of water on the implants was measured (at least 3 times) with a DGD Fast/60 contact angle meter and using WINDROP++ software. In the case of dry surfaces, the contact angles were measured by the 'water

droplet' method (CA $^{wd}$ ) at a fixed time of 30 sec with an average deviation of ±2°. The experimental values were thus representative of a solid/liquid interface ($\theta S_1$). The 'captive bubble' method ($CA^{cb}$) was used whenever the implant was hydrated (average deviation of ±3°). The contact angle, measured at a solid/gas interface (θb), was converted into a contact angle at a solid/liquid interface ($\theta S_2$) by equation 1.

$$\theta s_2 = 180° - \theta b \qquad [eqn.\ 1]$$

Comparison of θS, and $\theta S_2$ values for the same intraocular lens implant provides information on how hydration impacts the surface hydrophilicity/hydrophobicity of the lens implant.

The contact angle values for the dried samples were designated as $\theta S_1{}^{ns}$ before sterilization and $\theta S_1{}^{S}$ after sterilization. Similarly, $\theta S_2{}^{ns}$ and $\theta S_2$ referred to the captive bubble values for non-sterilized and sterilized hydrated samples, respectively *(cfr intra).*

*Table 1: Contact angle data for dry and hydrated plasma treated and PFDI-grafted treated hydrogel lens implants before and after sterilization.*

| Entry | Sample name | CA $^{wd}$ non-sterilized $\theta_{sl}{}^{ns}$ | CA $^{cb}$ non-sterilized $\theta_{s2}{}^{ns}$ | CA $^{cb}$ sterilized $\theta_{s2}{}^{s}$ | CA $^{wd}$ sterilized $\theta_{s1}{}^{s}$ |
|---|---|---|---|---|---|
| A | Unmodified [polished (A) and non-polished (A')] | 66° | 28° | 26° | 65° |
| B | PFDI grafted (polished) | 115° | 79° | 52° | 95° |
| C | RF Fl. 5'pol. | 84° | 28° | 24° | 68° |
| Entry | Sample name | $CA^{wd}$ non-sterilized $\theta_{s1}{}^{ns}$ | $CA^{cb}$ non-sterilized $\theta_{s2}{}^{ns}$ | $CA^{cb}$ sterilized $\theta_{s2}{}^{s}$ | $CA^{wd}$ sterilized $\theta_{s1}{}^{s}$ |
| D | RF Fl. 10' pol. | 108° | 26° | 22° | 67° |
| E | RF Fl. 15' pol. | 125° | 29° | 23° | 69° |
| F | RF Fl. 20' pol. | 129° | 27° | 20° | 66° |
| G | MW Fl. 30" pol. 9cm | 105° | 35° | 36° | 70° |
| H | MW Fl. 60" pol. 9cm | 109° | 33° | 33° | 68° |
| I | MW Fl. 90" pol. 9cm | 108° | 34° | 34° | 71° |
| J | MW Fl. 120" pol. 9cm | 108° | 35° | 33° | 72° |
| K | MW F1. 30" non-pol. 9cm | 101° | 27° | 26° | 72° |
| L | MW Fl. 60" non-pol. 9cm | 103° | 30° | 22° | 70° |
| M | MW Fl. 90" non-pol. 9cm | 105° | 152° | 27° | 66° |

(continued)

| Entry | Sample name | $CA^{wd}$ non-sterilized $\theta_{s1}{}^{ns}$ | $CA^{cb}$ non-sterilized $\theta_{s2}{}^{ns}$ | $CA^{cb}$ sterilized $\theta_{s2}{}^{s}$ | $CA^{wd}$ sterilized $\theta_{s1}{}^{s}$ |
|---|---|---|---|---|---|
| N | MW F1. 120" non-pol. 9cm | 103° | 31° | 23° | 67° |
| O | MW Fl. 30" non-pol. 11 cm | 97° | 28° | 22° | 72° |
| P | MW Fl. 60" non-pol. 11 cm | 101° ° | 29° | 22° | 75° |
| Q | MW Fl. 90" non-pol. 11 cm | 105° | 27° | - | - |
| R | MW F1. 120" non-pol. 11cm | 104° | 30° | - | - |

The success of the PFDI-grafting on the non-sterilized dry lens implants was confirmed by an increase of $\theta s_1{}^{ns}$ by about 50°, i.e. from 66° to 115° as shown in Table 1, entries A and B.

As a rule, modification by fluorinated plasma (Table 1, entries C to R) triggered the same general effect on $\theta s_1{}^{ns}$. However, these contact angles increased with the application time of the radio-frequency plasma from 84° after 5 min up to 129° after 20 min, which suggests that either the thickness or the fluorine content (or both) increased with time at least for the first fifteen minutes. Beyond that time, the surface hydrophilicity did not significantly change.

In contrast, $\theta S_1{}^{ns}$ was independent of the treatment time (beyond 30 sec) when the lenses were treated by the microwave plasma (Table 1, entries G to R). Moreover, lower contact angles were measured compared to the radio-frequency plasma performed at times longer than 10 min. This probably results from the formation on the surface of a fluorinated top layer with limited thickness after 30 sec and does not further develop hydrophobicity upon longer treatment times, which is in accordance with the results reported by Hochart, F. et al. (1999. Appl. Surf Sci., 42(l-4), 574-578).

As the intraocular lens implants are hydrated in use, it is important to have the lenses equilibrated in water and to measure their contact angles of air by the 'captive bubble' technique.

The impact of hydration is straightforward. Indeed, although the air bubble is spreading on the PFDI-coated surface, it is no longer the case when the surface was treated by fluorinated radio-frequency- and microwave-plasma. This preliminary observation was confirmed by the experimental data. Indeed, the high hydrophilicity of the original non-modified hydrated intraocular lens implants decreased by 51° upon grafting of PFDI (Table 1, entries A and B), which is quite comparable to the effect noted for the previously dry lens implants.

In sharp contrast, $\theta S_2{}^{ns}$ remained substantially unchanged upon treatment with fluorinated-plasma (Table 1, entries C to R). There may be two possible events that might lead to this loss of hydrophobicity, the extent of which was investigated (*cfr infra*). One is related to extraction of some non-covalently grafted fluorinated segments from the implant surface, and the other possibility may be the reorganization of small fluorinated segments from an unstable top layer of the fluorinated surface upon water-swelling.

Sterilization of the unmodified hydrated lens implants and their polishing did not affect the contact angles.

As a rule, the hydrophobicity of the fluorinated dry samples ($\theta s_1{}^{ns}$ and $\theta_{s1}{}^{s}$) in Table 1, entries B to R) decreased upon sterilization (at standard conditions - 120°C, 1.5 bar for 21 min), to the point when the contact angle for the original lens implants was approximately restored to the values of the sterilized unmodified lens implants by fluorinated-plasma treatment. The exposure of the samples to water more likely triggers the extraction of non-covalently grafted fluorinated chain segments and the reorganization of mobile covalently grafted fluorinated chain segments at the surface.

Sterilization of the hydrated lens implants, non-treated and treated by fluorinated radio-frequency and microwave plasma ($\theta s_2{}^{ns}$ and $\theta s_2{}^{s}$ in Table 1), has no significant impact on their surface hydrophilicity, it may be that the chain segments reorganized themselves during water-swelling before sterilization.

A 27° diminution of the surface hydrophobicity of PFDI-grafted lens implants was shown after sterilization ($\theta_{S2}{}^{s}$ in Table I , entry B). However, the resulting surface still remained a significant 26° more hydrophobic than the original untreated lens implant, signifying the presence of stable, grafted, PFDI-chains, which resist hydration and sterilization conditions.

Two phenomena might be responsible for a small loss of hydrophobicity for PFDI-grafted lens implants, namely extraction of non-covalently grafted PFDI-chains or their reorganization at the level of the upper layer of the soft (hydrated) substrate, the latter being favoured by the increased pore size of the cross-linked hydrogel at the elevated sterilization temperature (Hoffman, A.S. 2001. Annals New York Acad. Sci., 944, 62-73).

The impact of such pores was estimated by slowly drying the hydrated and sterilized lens implants, which was expected to result in re-exposure of the PFDI-chains to the surface of the implant, which have possibly penetrated the upper layer of the implant. To investigate this, the hydrated and sterilized PFDI-grafted lens implant was first exposed to air for 24 h, followed by completely drying at 50 °C for 2 days in vacuo. Its contact angle of water ($\theta s_1^s$ in Table I , entry B) was then measured by the 'water droplet' method and was found to be 30° higher compared to the original, untreated lens implant (Fig. 3), which is comparable to the result obtained for the hydrated sterilized surfaces ($\theta S_2^s$). This similarity suggests that no significant number of PFDI-chains are re-exposed to the surface and, thus, they are either not at the surface at all (i.e. they have been extracted), or they remain trapped in the gel pores upon drying. XPS measurements (discussed below) of this dried lens implant (Table 2, entry B, sterilized) revealed diminution of the fluorine content compared to the same surface before hydration and sterilization. If there were any PFDI-chains non-exposed to the lens implant surface but present in the pores of its upper layer, they should have been detected at the XPS probing depth of 10 nm and the initial O/F atomic ratio of 1/2.67 should have been preserved. Since it is not the case, it might be concluded that, the loss of hydrophobicity during sterilization is due to extraction of non-covalently grafted PFDI-chains, rather than reorganization of the soft (hydrated) surface.

***X-ray photoelectron spectroscopy (XPS) data***

Additional information on the coating thickness was researched by XPS. XPS was performed with SSX-100 spectrometer (Surface Science Instruments), equipped with a monochromatic Al K$\alpha$ source and a spherical sector analyser with a multichannel detector. The energy resolution was defined as the full width at half-maximum (FWHM) of the Ag 3d5/2 peak (0.65 eV). All the spectra were collected in the normal emission mode with an X-ray spot of 1000 $\mu$m. A nickel grid was mounted 1-2 mm above the sample, which was also flooded with a wide beam of low-energy electrons, in order to prevent the surface from being electrostatically charged.

*Table 2: XPS data for representative plasma and chemically fluorinated, coatings before and after sterilization. Sample codes correspond to sample codes from table 1.*

| Entry | Sample name | O/ C/ F experimental atomic ratio by XPS | | | | | | | |
|---|---|---|---|---|---|---|---|---|---|
| | | Non-sterilized | | | | Sterilized | | | |
| | | O | C | F | N | O | C | F | N |
| A | Unmodified | 1 | 2.6 | 0 | 0 | 1 | 2.6 | 0 | 0 |
| B | PFDI grafted | 1 | 2.06 | 2.67 | 0.14 | 1 | 2.42 | 1.87 | 0.11 |
| C | RF Fl. 5' pol. | 1 | 3.27 | 2.33 | 0 | 1 | 3.18 | 0.14 | 0 |
| D | RF Fl. 10' pol. | 1 | 3.14 | 2.86 | 0 | 1 | 3.23 | 0.06 | 0 |
| E | RF Fl. 15' pol. | 1 | 3.77 | 3.42 | 0 | 1 | 1.9 | 0.24 | 0 |
| F | RF Fl. 20' pol. | 1 | 4 | 3.91 | 0 | 1 | 3.29 | 0.2 | 0 |
| I | MW Fl. 90" pol. 9cm | 1 | 3.24 | 1.4 | 0 | 1 | 3.94 | 0.19 | 0 |
| J | MW Fl. 120" pol. 9cm | 1 | 3.14 | 1.2 | 0 | 1 | 3.74 | 0.19 | 0 |

Dramatically decreased surface hydrophobicity was shown for the dry radio-frequency plasma treated lens implants after hydration and sterilization. As discussed above, the contact angles of water obtained by the 'water droplet' method for these implants ($\theta S_1^s$ in Table I , entries C to F) were reminiscent of the contact angles ($\theta S_1^s$) of the original unmodified substrate and were significantly smaller than the ones for the same lens implants before hydration and

sterilization. This is likely to be due to extraction of the fluorinated segments from the top surface layer. This is also supported by the XPS measurements performed for the same lens implants (Table 2, entries C to F, sterilized), for which strong diminution of the fluorine content was detected. It is not surprising that some fluorine is reported for these implants, which would result from fluorinated segments remaining trapped in the top surface layer upon drying (*cfr infra*). However, these trapped segments do not significantly impact on the surface hydrophobicity.

The situation slightly differs when fluorinated microwave-plasma treated implants (polished or not) are concerned. The same drying procedure of the hydrated sterilized lens implants resulted in surfaces ($\theta S_1^s$ in Table 1, entries G to R), which are ~30° less hydrophobic than before hydration and sterilization ($\theta S_1^{ns}$), but ~5° (with a few exceptions) more hydrophobic than the unmodified original implant ($\theta S_1^s$). Stably grafted fluorinated segments, might be responsible for this and are probably exposed to the surface, thus directly altering its hydrophobicity, in contrast to the dried sterilized radio-frequency plasma treated lens implants, since the amount of fluorine detected by XPS for radio-frequency and microwave plasma treated lens implants was similar (Table 2, entries I and J, sterilized).

Hydrated samples were also analyzed by Atomic Force Microscopy with a PicoPlus 5500 equipped with a fluid cell (Agilent Technologies) operated at room temperature in the intermittent contact mode. Pointprobe® Plus Silicon cantilevers (Nanosensors) were used (nominal resonance frequency of 330 kHz, force constant of 42N/m.

It must be recalled that a common use of plasma is the cleaning of solid surfaces. However, wherever the treatment is extended beyond the elimination of surface contaminants, the etching of the surface is the predominant effect (Bogaerts et al. 2002 Spectrochemica Acta Part B 57: 609-658). For instance, oxygen- and fluorine-containing plasmas are frequently used for etching the surface of polymeric materials (Chan et al. 1996 Surf. Sci. Reports 24: 1-54).

Surface roughness is known to have a direct influence on protein and cell repulsion, thus attention was paid to the impact of the IOLs surface treatment on the surface topography. The surface of hydrated lenses was thus observed by AFM equipped with a liquid-water-cell. Firstly, the surface of the polished original lenses was observed before (Fig. 4-A; Fig. 5A) and after fluorination by RF plasma for 5 min, 10 min and 15 min (Fig. 4C,D and E; Fig. 5A). After 5 min, a decrease of the the root-mean-square (RSM) roughness of the surface was reported and was consistent with a cleaning effect of the plasma. This initial step was however followed by surface etching upon increasing the treatment time. The same general effect was previously reported by Chan et al (1996 Surf. Sci. Reports 24: 1-54), who explained that surface reactions, etching, and plasma polymerization can occur simultaneously in fluorine containing plasma, the predominant reaction depends on the polymer substrate, the gas feed and the operating conditions. However, in saturated fluorocarbon plasmas, the fluorine atoms are non-polymerizable etching species, such that etching dominates rapidly in case of precursors with F/ C atomic ratio higher than -2.

As a rule, the surface topography of the polished lenses is more affected by the RF plasma (13.56 MHz) (Fig. 4C-E; Fig. 5A) than by the MW plasma (2.54 GHz) (Fig. 4G-J; Fig. 6, 9 cm apart from the plasma source) at least within the limits of the experimental conditions used (distance from the plasma source and treatment time). Even treated by MW plasma for the longer time of 120 sec, the RMS roughness of the originally polished surfaces decreased, which suggests that cleaning basically dominates etching under these conditions. According to Denes et al. (2004 Progr. Polym. Sci 29: 815-885], a key criterion is the capacity of the plasma discharges to fill up the entire reactor, thus to impact the substrate whatever the distance from the plasma source. These authors explained that RF discharges most often fill the entire reactor, whose dimensions are usually smaller than the wavelength of the RF field (13.56 MHz roughly corresponds to 22 m). In contrast, the efficiency of MW plasmas strongly depends on the sample position in the plasma chamber, the wavelengths being 12.24 cm at 2.54 GHz. Consistently, these authors reported a more effective etching of a cellophane surface when treated by a pulsed hydrazine plasma at 40 kHz rather than at 13.56 MHz.

Although the implanted lenses are previously polished, it would be nice to bypass this preliminary step, which would be possible if the surface was simultaneously polished and fluorinated by the plasma. This opportunity was tested with the MW plasma which is less aggressive towards the surface than the RF plasma. The conditions used for the plasma treatment of sample ***H*** (polished) reported in Figure 4 were extended to originally non-polished IOL. After treatment by MW plasma for 60 sec, the general furrow-like aspect of the surface observed by AFM did not change (Fig. 4A' and 4L). Nevertheless, the RMS roughness decreased without however being as small as for the unmodified polished surface (Fig. 5B). This observation was also valid when the surface-plasma source distance was increased from 9 to 11 cm (Fig. 5B). Even at longer MW plasma treatment times (up to 120 sec), the roughness of the fluorinated non-polished samples (Fig. 4O to 4R; Fig. 6) did not exceed that one of the originally non-polished surface, and the furrow-like aspect was preserved. This signifies lower impact of the fluorinated MW plasma on the surface topography, compared to the fluorinated RF plasma, at least at the conditions investigated.

Although not particularly relevant to the invention, non-polished IOLs were also treated by MW argon plasma for 30, 60, 90 and 120 sec. The main observation (Fig. 6, A and B) is that this type of plasma is more etching-effective than the fluorinated MW plasma under the comparable conditions and the general aspect of the surface was seriously affected with furrows no longer observed even after 30 sec (Fig. 4- T to V). The same conclusion was drawn elsewhere (Raffaelo-Addamo et al. 2006 Appl. Surf. Sci 252: 2265-2275; Polleti et al. 2003 Appl. Surf. Sci. 219: 311-316), e.g. with a poly(ethylene terephthalate) substrate.

Finally, the surface topography of hydrated lenses was found to be slightly rougher as result of the wet chemistry PFDI- grafting (Fig 7), because of the formation of rather large non-uniformly distributed protuberances (Fig. 4- B). This phenomenon might be explained with intermolecular reaction of the bifunctional PFDI during the lens modification, as a result of traces of humidity present in the reactor.

***Water swelling ability***

It was investigated whether the formation of a fluorinated coating/modification on the surface of hydrophilic intraocular lens implant, would affect the water-swelling ability of the implants, since a water-non-permeable barrier may be formed. The plasma treated samples were surface-modified only on one side, leaving the other side non-modified. Thus, water-swelling ability should not be affected.

Water uptake (W%) was determined according to equation 2.

$$W\% = [(m_{\text{hydrated}} - m_{\text{dry}})/ m_{\text{hydrated}}]*100 \qquad [\text{eqn. } 2],$$

where:

- m hydrated is the weight of the sample in the fully hydrated state (swelling for at least 48 h at room temperature in a water-filled sealed pot). The non-absorbed water from the surface of each sample was removed by filter paper just before weighing; and

- $m_{dry}$ is the weight of the same sample dried for 48 h at 50 °C under reduced pressure.

Water uptake of 26 % was measured for all of the treated lens implants, which was equal to the water uptake of untreated lens implants.

In the case of the wet-chemical PFDI-coating, the entire surface of the lens implants was modified, thus, modulation of the water-swelling ability of the implant may be expected. The fluorinated coating/modification could be non-water-permeable if it had absolute integrity, which is unlikely. Water-swelling of the PFDI-grafted lens implants did occur, although with a rate slower than that of the un-modified and the plasma treated lenses, and resulted in 26% water uptake within two days, similar to the un-treated lens implants. Therefore, the PFDI-grafting did not affect the water-swelling ability of the lens implants.

***Lens epithelial cell adhesion: in vitro testing***

It is usually accepted that posterior capsular opacification does not occur in two extreme situations, i.e. when the lens implants exhibit very strong or very poor cell repellency (Denes, F.S. et al. 2004. Prog. Polym. Sci. 29, 815-885). Where the implant surface is cell repellent, lens epithelial cells have no or very poor chance to adhere on the posterior face of the intraocular lens implant and posterior capsular opacification is prevented from occurring.

According to Linnola, R. (2001. Academic dissertation. *Oulun Yliopisto,* Oulu), whenever cells strongly adhere to a lens implant, a monolayer bridge of lens epithelial cells might be built between the implant and the posterior capsule. This 'sandwich' pattern is strong enough to impede further cell ingrowth.

Lens epithelial cell growth and adhesion on the surface of fluorinated-plasma treated and PFDI- grafted lens implant was investigated by *in vitro* cell adhesion testing with lens epithelial cells.

***Lens epithelial cell isolation and culturing***

Lens epithelial cells (LECs) were isolated from the lens crystalline capsule of pig eyes (Pietrain-Landrace pig; from the "Abattoir communal de Liège") and cultured as reported elsewhere (Dumaine, L. 2004. Thesis. Ecole Centrale de Lyon, 79-85; Bozukova, D. et al. 2007. Biomacromolecules, 8, 2379-2387; Bozukova, D. 2008. Langmuir, 24, 6649-6658). Cells in the $4^{th}$ to $10^{th}$ passage were used for testing.

***Lens epithelial cell adhesion: in vitro test conditions***

The test was similar to the one reported in Bozukova, D. et al. (2007. Biomacromolecules, 8, 2379-2387) and

Bozukova, D. (2008. Langmuir, 24, 6649- 6658). The lens implant samples were pre-conditioned in physiological solution (SERAG BSS©, pH 7.3) for 2 days and cut down to disks with a 6 mm diameter, in order to fit the lens implants into the test wells. The lens implants were washed and sterilized in a physiological solution at 1.5 bar, 120 °C, for 21 min. The lens implants were then fixed to the bottom of a 96-well plate and conditioned with culture medium (CM, 87% Dulbecco's Modified Eagle's Medium (Cambrex), 10% bovine foetal serum (Gibco), 1% of antibiotics (penicillin/streptomycin 10 000 U/ 10 000 μg/ml, 1 % glutamine and 1 % HEPES) in an incubator for 2 h. After removal of the culture medium, a 7500 cells/ 100 μL suspension was added to each well followed by 100 μL culture medium. Three days later, the culture medium was removed, and the lens implants were carefully washed with PBS in order to eliminate the non-adhering and dead cells. The adhering cells were fixed with 4% paraformaldehyde at 4 °C for 2 h, washed three times with PBS and kept in PBS at 6 °C.

The surface of the lens implants were observed with an optical microscope Zeiss, Axioplan, equipped with Epiplan-NEOFLUAR objectives (5x magnification) and a CCD camera (model ICD-42E). The images were visualized with XnView software. Each sample was analyzed in triplicate. The surface area occupied by the cells was determined with Dpx View Pro Image management software.

A 3 day period of cell-surface contact was found to be appropriate for the monitoring of the biological response of the surfaces. Indeed, this period of time is long enough for cells to attach, grow and proliferate, if they do so, and a short enough period to not significantly change the culture medium, which could result in the loss of poorly or non-adhering cells.

In each series of testing, an unmodified lens implant was used as an initial reference. The lens epithelial cells used were in $4^{th}$ to $10^{th}$ passage, which may result in differences in their adhesion to the lens implant as result of the higher adaptability of cells with larger number of passage to the testing conditions. For this reason, attention was paid to the percentage of the surface occupied by cells (Fig. 8) rather than to the details of the occupation shown by optical microscopy.

***Lens epithelial cell adhesion: in vitro test results***

The chemical grafting of PFDI onto the lens implants proved to be a more effective fluorination technique than the plasma methods, leading to a more hydrophobic and stable coating. Cell adhesion was decreased by at least a factor of 2 compared to the unmodified surface (Fig. 8- C), which highlighted the role of the surface hydrophobicity of the hydrated lens implants (see $\theta_b^{ns}$ in Table 1).

Compared to the unmodified surface, treatment by fluorinated radio-frequency and microwave plasmas made the surface more adhesive towards lens epithelial cells (Fig. 8, A and B). Moreover, cell adhesion increased with the treatment time until a plateau value of -75 % for fluorinated microwave plasma, and 95% for fluorinated radio-frequency plasma of the total surface was reached.

In order to take into account a possible role of the surface roughness on the biological response of the treated surfaces, the *in vitro* lens epithelial cell adhesion test was also conducted with lens implants exposed to microwave argon plasma as a control experiment. Microwave argon plasma treatment of the lens implant surface provides an etched non-fluorinated surface. Microwave argon plasma treatment is also more effective at etching the surface than radio-frequency and microwave plasma fluorination. Figure 8-B shows that the more effective etching of a non-fluorinated surface has only a negligible influence on the cell adhesion, at least in the limits of roughness considered herein.

Thus, the proper fluorination of intraocular lens implant, which is possible by the chemical grafting of poly (perfluroalkyl ether) oligomers, can make the lens implants cell repellent, which is important in reducing levels of posterior capsular opacification that can occur after implantation.

***Optical performances of the modified lens implants***

Even though fluorination of intraocular lens implants provides them with the desired biological activity, this strategy would be unacceptable if the optical transparency was adversely affected. Therefore, the transparency of the lens implants modified by plasma and chemical grafting (hydrated by Milli-Q water) was measured with a Perkin Elmer UV/Vis spectrophotometer Lambda 14P and a quartz cell. For Diopter measurement, the intraocular lens implants were preconditioned in a physiological solution and measured with a ROTLEX IOLA™ instrument.

The transparency of all modified lens implants was systematically higher than 90% at wavelengths higher than 400 nm (Fig. 9). The strong absorption below 350 nm must be assigned to the UV-blocking agent added to the commercially available intraocular lens implants.

The observation that the transparency is preserved for the plasma treated lens implants is not surprising on account of the poor stability and partial loss of the fluorinated materials upon hydration and sterilization of the lens implants (Fig. 9A and 9B).

In spite of the effective grafting of PFDI, the transparency of the lens implants is maintained (Fig. 9- C), as well.

The diopter (the relative power) of the hydrated intraocular lens implants, was also maintained upon grafting of PFDI. Therefore, fluorination of the intraocular lens implant surface by chemical grafting is a valuable strategy for restricting cell adhesion and thus posterior capsular opacification occurrence while preserving the optical performances of the original lens.

***Sliding ability***

Sufficient sliding ability of the fluorinated intraocular lens implant is another desired property, because during implantation the implants are folded, and then inserted through an approximately 2.4 mm incision and finally they are required to slip into the capsular bag. Should the surface of the lens implant be sticky, the insertion would be difficult and would be harmful to the eye. Therefore, the PFDI-grafted and fluorinated radio-frequency plasma treated hydrated intraocular lens implants were tested by handling the implants with an intraocular lens implant injecting device currently available on the market place (MiniSet Injection Pack and an incision ≥2.4 mm (PhysIOL SA, Belgium). The injector was pre-loaded with a hydrated intraocular lens implant, which was then pushed out.

Again, the PDFI-grafted lens implant offered a decisive advantage, with only the intraocular lens implants modified by PFDI-grafting having sufficient sliding ability for proper implantation.

**CONCLUSIONS**

Two strategies were investigated and compared (i.e. exposure to radio-frequency and microwave plasmas and surface grafting of a perfluoropolyether by wet-chemistry) for making the surface of a poly(HEMA-*co*-MMA) intraocular lens implants hydrophobic by fluorination. Plasma fluorination was found not to be an acceptable method because of the poor stability of the modified top layer, which does not resist water-swelling and sterilization of the implants. The final hydrophobicity is also too low for the surface to be cell repellent.

Extensive enough fluorination was carried out by wet-chemical grafting of perfluorinated oligomers (PFDI). The grafted chains withstand not only the water-swelling but more importantly sterilization of the implants. Moreover, the perfluorinated oligomers provide a surface with sufficient lens epithelial cell repellency, they are not detrimental to the optical performance of the implants, and they offer an improved sliding ability for implantation of the implants.

**Claims**

**1.** A method for modifying the surface of a material for use with a biological sample or tissue comprising the steps:

- providing a material having a surface which comprises reactive-functional groups;
- providing fluorinated molecules having reactive-functional groups complimentary to those on the material surface;
- using wet chemistry to attach the fluorinated molecules to the surface of the material by reacting the reactive-functional groups of the implant with the complementary reactive-functional groups of the fluorinated molecules.

**2.** The method according to claim 1, wherein the fluorinated molecules form a brush of fluorinated molecules on the material surface.

**3.** A method for modifying the surface of a material for use with a biological sample or tissue comprising the steps:

- providing a material having a surface which comprises reactive-functional groups;
- providing fluorinated molecules having reactive-functional groups complementary to those on the material surface;
- attaching the fluorinated molecules to the surface of the material by reacting the reactive-functional groups on the material with the complementary reactive-functional groups of the fluorinated molecules, to form a brush of fluorinated molecules on the surface of the material.

**4.** The method according to claim 3, wherein attaching the fluorinated molecules is achieved by a wet-chemical reaction.

**5.** The method according to any preceding claim wherein the material is used in an implant, a medical or surgical device or instrument, or a cell or culture vessel.

**6.** The method according to any preceding claim, wherein the fluorinated molecules are homomeric or copolymers of

two or more different monomers.

7. The method according to any preceding claim, wherein the fluorinated molecules are hydrophobic oligomers or polymers

8. The method according to any preceding claim, wherein the fluorinated molecules are perfluorinated polyethers.

9. The method according to any preceding claim, wherein the fluorinated molecules are of the Formula I:

Formula I: 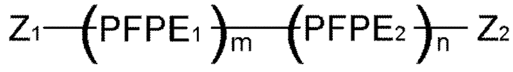

$PFPE_1$ is 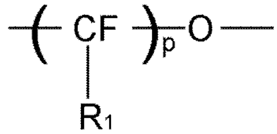

$PFPE_2$ is 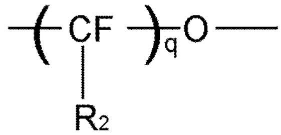

$Z_1$ is a reactive functionality, which might be, but is not limited to, - CH2=CHCOO-, CH2=C(CH3)COO-, oxirane, alcyne, anhydride, -CHO, - NH2, -COOH, -OH, -COCl, -NCO, -COBr, -CN, -N3,...
$Z_2$ might be either-H; -F; -$CH_3$; -$CF_3$; -$CF_2H$; -$CFH_2$,
or a reactive functionality, which might be, but is not limited to, CH2=CHCOO-, CH2=C(CH3)COO-, oxirane, alcyne, anhydride, -CHO, - NH2, -COOH, -OH, -COCl, -NCO, -COBr, -CN, -N3,...
$R_1$ is H, F, $CH_3$, $CF_3$, $CHF_2$,$CH_2F$;
$R_1$ is H, F, $CH_3$, $CF_3$, $CHF_2$,$CH_2F$;
m and n might have the same values, or different; and are equal or greater than 1; or not more than one might be equal to 0, the other one being equal or greater than 1;
p and q might have the same values, or different; and are between 1 and 5.
$PFPE_1$ and $PFPE_2$ in Formula I may be the same or different, and may be randomly distributed or as blocks throughout the chain.

10. The method according to any preceding claim, wherein the reactive-functional groups on the implant surface are selected from any of the group comprising -NH2, -COOH, -OH, -COCl, -NCO, -COBr, -CN, and -$N_3$ or combination thereof, and the complementary reactive-functional groups of the fluorinated molecules are selected from any of the group comprising - $CH_2$=CHCOO-, $CH_2$=C($CH_3$)COO-, oxirane, alcyne, anhydride, -CHO, - $NH_2$, -COOH, -OH, -COC1, -NCO, -COBr, -CN, and -$N_3$, or combinations thereof.

11. The method according to any preceding claim, wherein the material forms a part of all of an intraocular lens implant.

12. The method according to any preceding claim, wherein the material is selected from any of the group comprising silicone polymers; hydrocarbon polymers; hydrogels; acrylic polymers; polyesters; polyamides; polyurethanes; silicone polymers with hydrophilic monomer units; metal; and glass; or combinations thereof.

13. A material having a surface modification provided by the method of any preceding claim.

14. An implant comprising a material according to claim 13.

15. A method of reducing or preventing posterior capsular opacification of an IOL implant comprising modifying a surface of an IOL implant by applying the method of the invention to the implant.

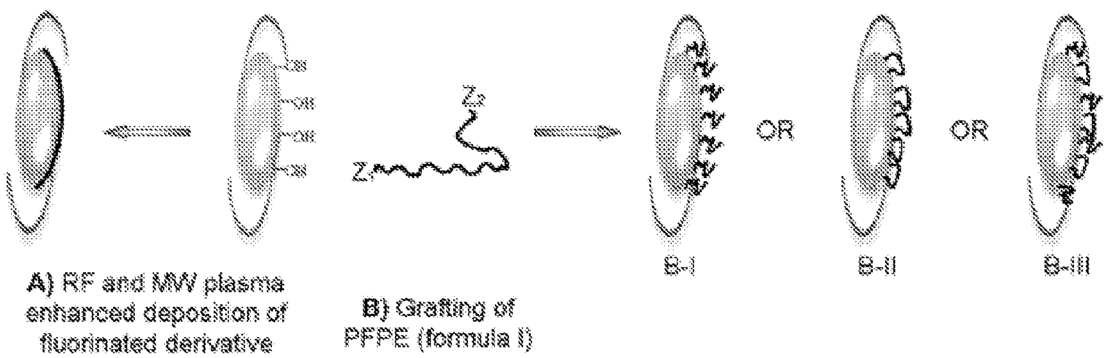


Figure 1

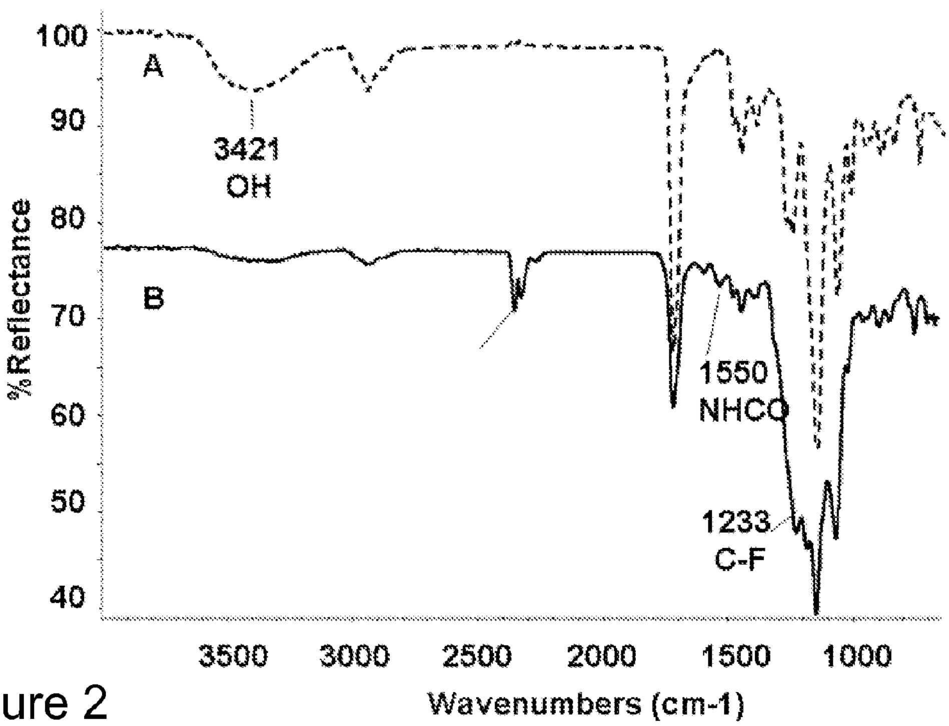


Figure 2

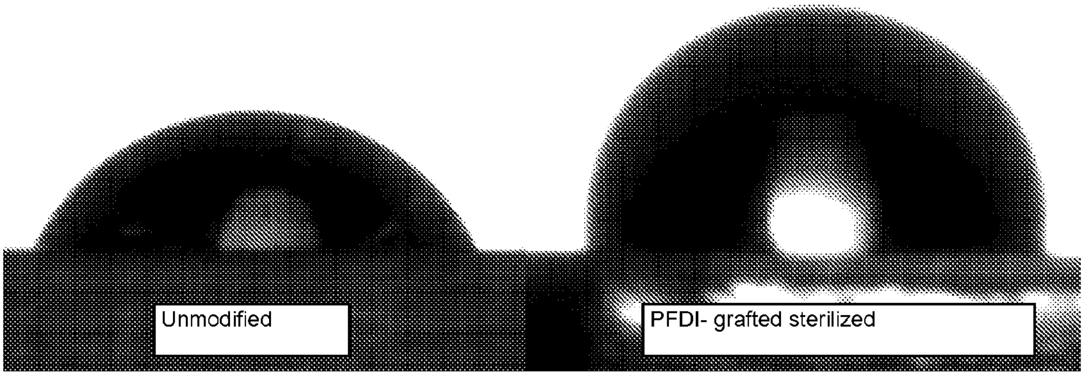


Figure 3

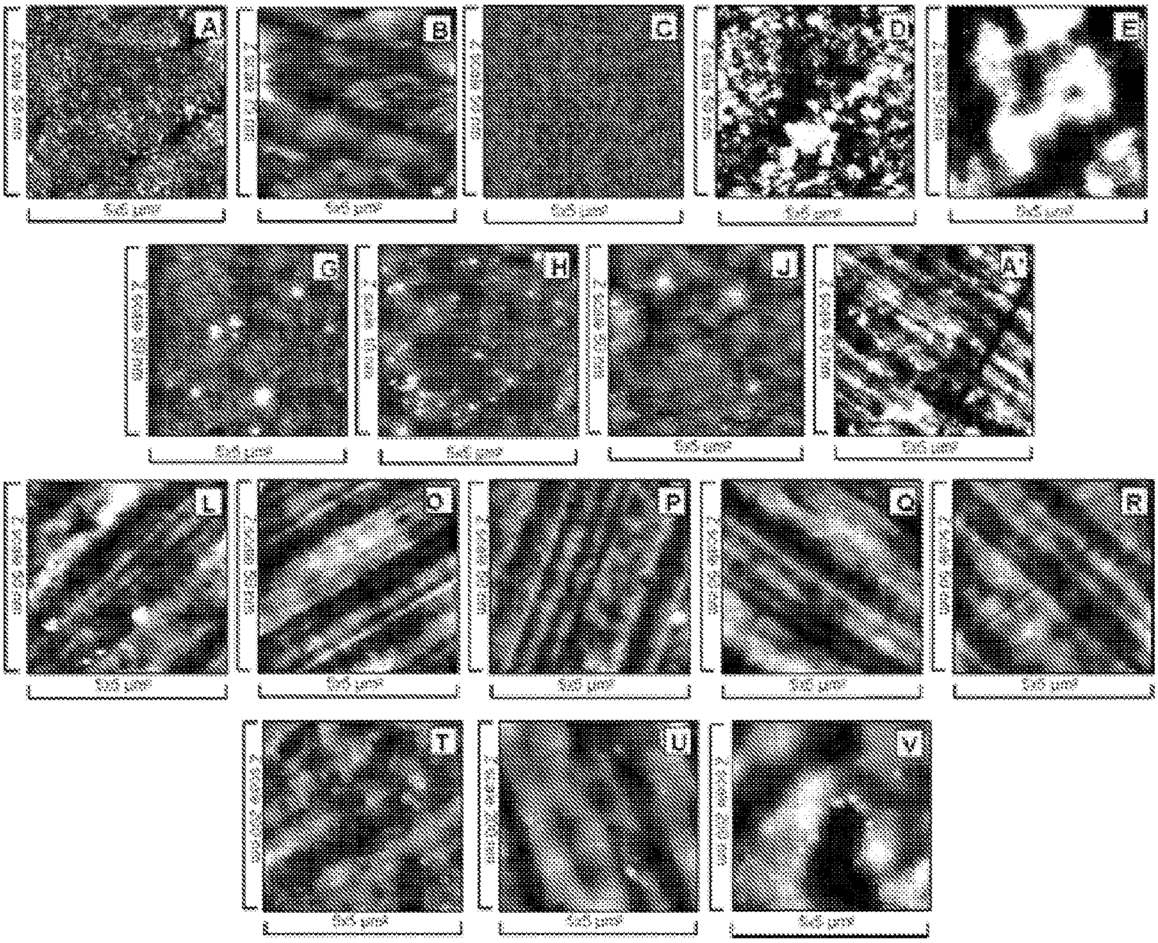


Figure 4

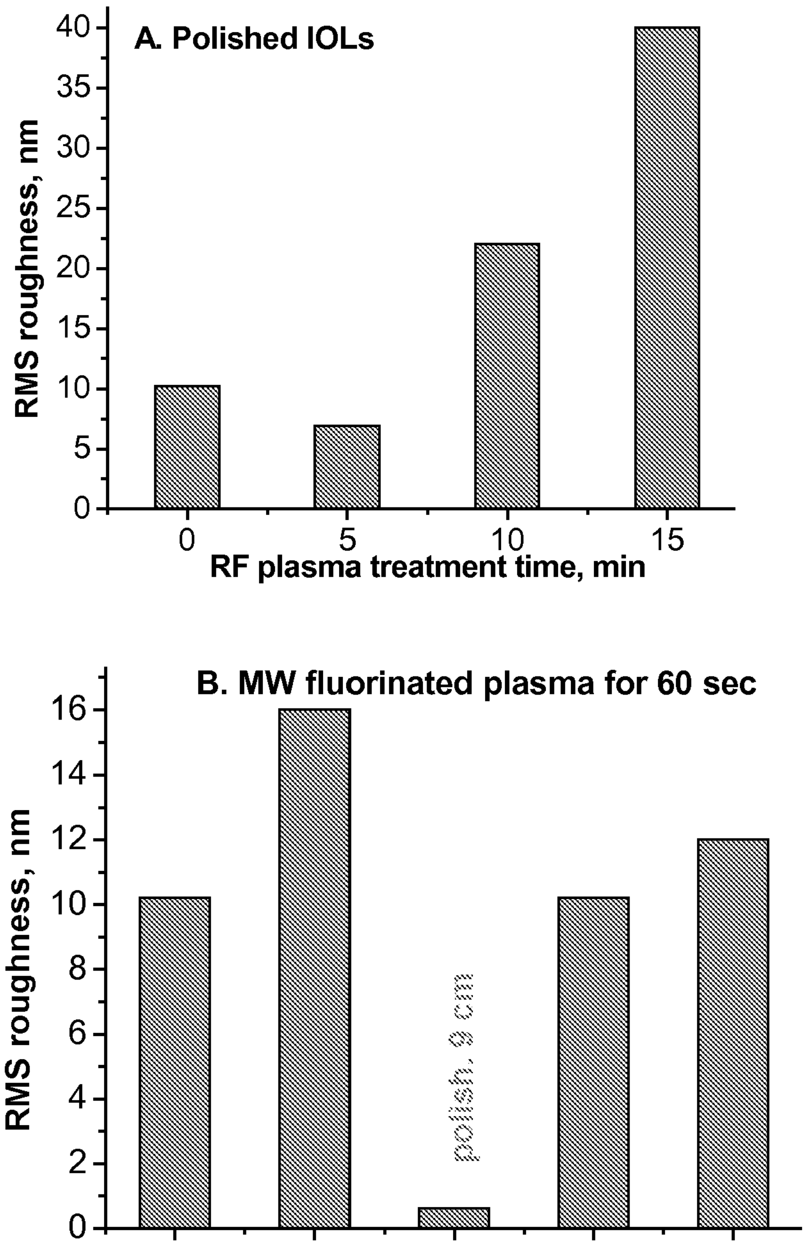
A. Polished IOLs
RMS roughness, nm
0
5
10
15
20
25
30
35
40
0
5
10
15
RF plasma treatment time, min
B. MW fluorinated plasma for 60 sec
RMS roughness, nm
0
2
4
6
8
10
12
14
16
polish, 9 cm

Figure 5

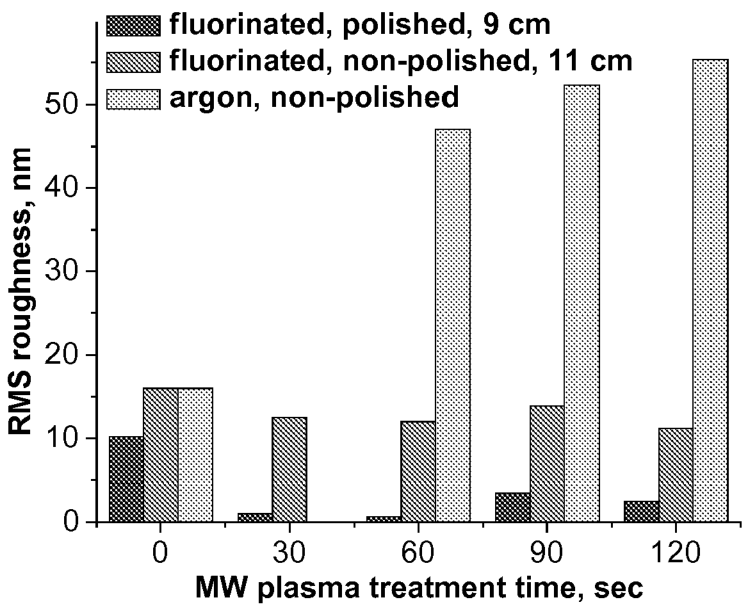


Figure 6

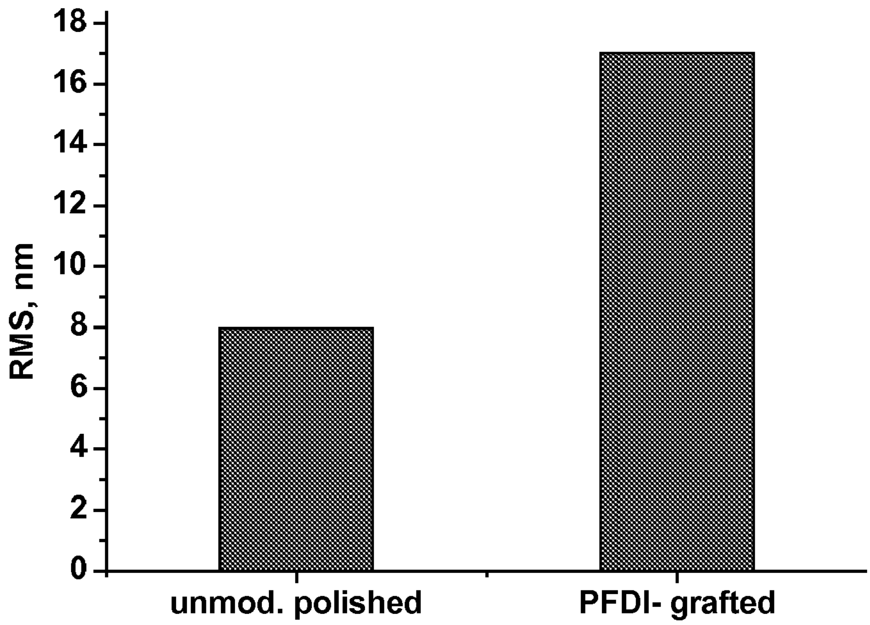


Figure 7

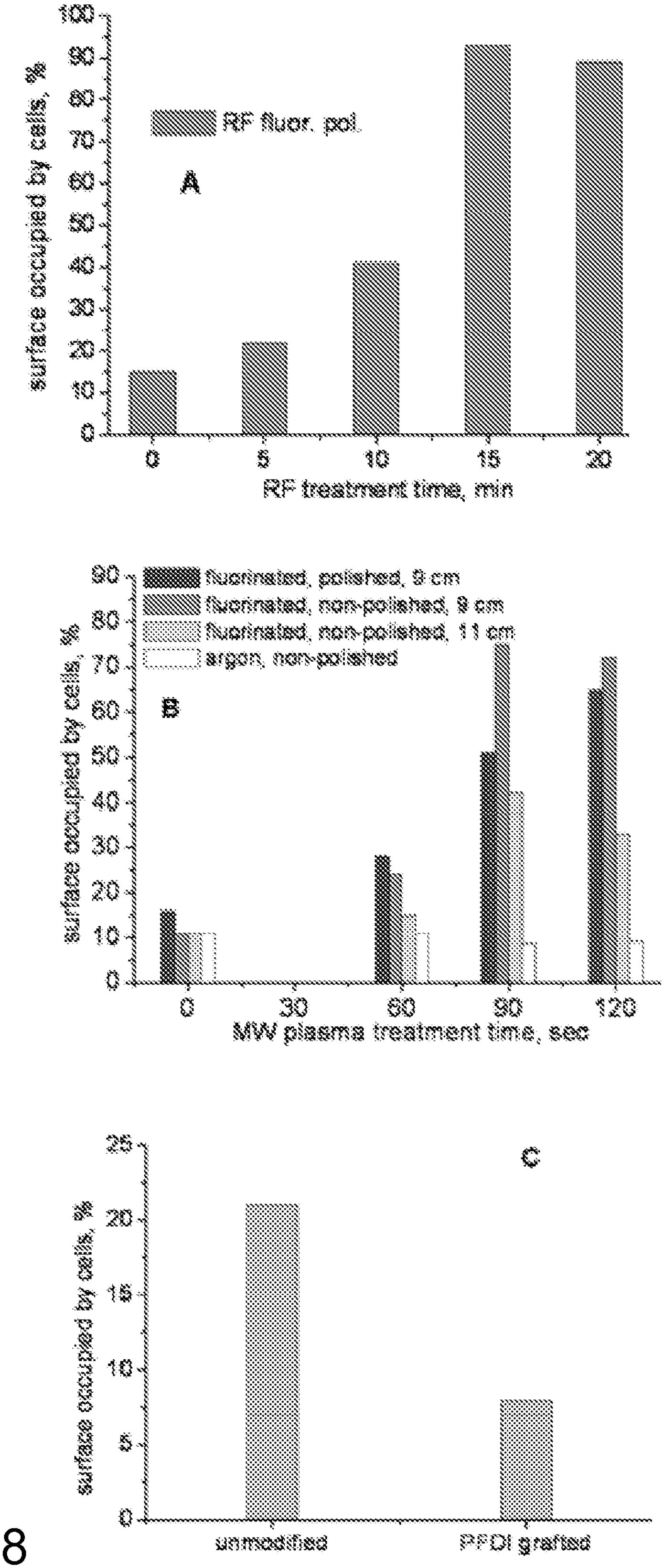
100
90
80
70
60
50
40
30
20
10
0
surface occupied by cells, %
RF fluor. pol.
A
0
5
10
15
20
RF treatment time, min
90
80
70
60
50
40
30
20
10
0
surface occupied by cells, %
fluorinated, polished, 9 cm
fluorinated, non-polished, 9 cm
fluorinated, non-polished, 11 cm
argon, non-polished
B
0
30
60
90
120
MW plasma treatment time, sec
25
20
15
10
5
0
surface occupied by cells, %
C
unmodified
PFDI grafted

Figure 8

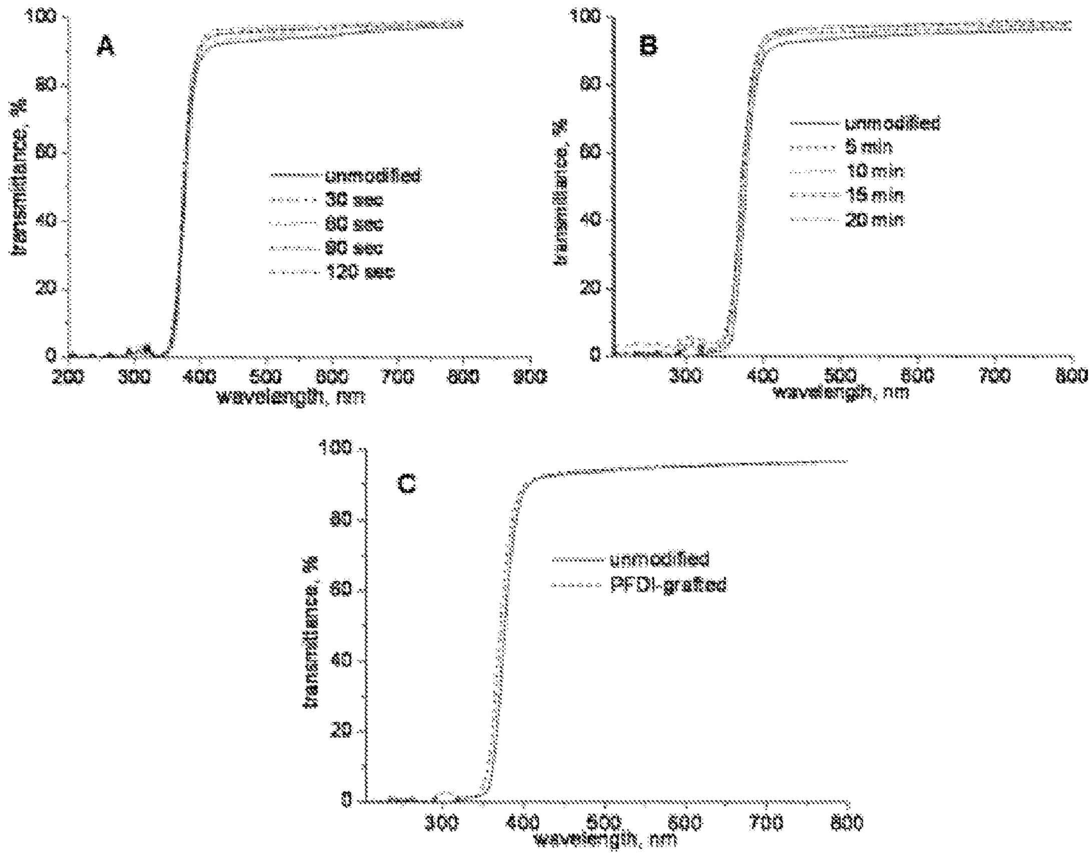
A
transmittance, %
wavelength, nm
unmodified
30 sec
60 sec
90 sec
120 sec
B
unmodified
5 min
10 min
15 min
20 min
C
unmodified
PFDI-grafted

Figure 9

Europäisches Patentamt
European Patent Office
Office européen des brevets

# EUROPEAN SEARCH REPORT

Application Number
EP 09 15 8848

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/160139 A1 (HUANG JIANG [US] ET AL) 31 October 2002 (2002-10-31)<br>* paragraph [0096] - paragraph [0106] *<br>* paragraph [0108] *<br>* paragraph [0128] - paragraph [0130] *<br>----- | 1-7, 12-14 | INV.<br>C08J7/12<br>A61L27/34<br>A61F2/16 |
| X | US 2004/006386 A1 (VALINT PAUL L [US] ET AL VALINT JR PAUL L [US] ET AL)<br>8 January 2004 (2004-01-08)<br>* paragraph [0007] *<br>* paragraph [0016] - paragraph [0021] *<br>* examples 6-11,20,21,23 *<br>----- | 1,5,6, 10-15 | |
| X | US 2004/166236 A1 (MENZ DIRK HENNING [DE] ET AL) 26 August 2004 (2004-08-26)<br>* paragraph [0011] - paragraph [0022] *<br>* example 1 *<br>----- | 1-15 | |
| X | US 2008/248293 A1 (HANSON ERIC L [US] ET AL) 9 October 2008 (2008-10-09)<br>* paragraph [0005] - paragraph [0008] *<br>* paragraph [0017] - paragraph [0023] *<br>* paragraph [0041] - paragraph [0044] *<br>----- | 1-4, 6-10,12, 13 | TECHNICAL FIELDS SEARCHED (IPC)<br>C08J<br>A61L<br>A61F |
| L | BOZUKOVA, DIMITRIYA (PH.D. THESIS): "Strategies for improving the surface and bulk performance of poly(2-hydroxyethyl methacrylate -co- methyl methacrylate) hydrogel intraocular lenses"<br>INTERNET ARTICLE, [Online]<br>23 January 2009 (2009-01-23), XP002551209<br>CERM, UNIVERSITY OF LIÈGE<br>Retrieved from the Internet:<br>URL:http://orbi.ulg.ac.be/handle/2268/25426> [retrieved on 2009-10-20]<br>* document not available online; relevance unknown *<br>* abstract *<br>----- | 1-15 | |

The present search report has been drawn up for all claims

| Place of search | Date of completion of the search | Examiner |
|---|---|---|
| The Hague | 20 October 2009 | Lichau, Holger |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 09 15 8848

This annex lists the patent family members relating to the patent documents cited in the above-mentioned European search report.
The members are as contained in the European Patent Office EDP file on
The European Patent Office is in no way liable for these particulars which are merely given for the purpose of information.

20-10-2009

| Patent document cited in search report | Publication date | Patent family member(s) | Publication date |
|---|---|---|---|
| US 2002160139 A1 | 31-10-2002 | US 2006093836 A1 | 04-05-2006 |
| US 2004006386 A1 | 08-01-2004 | AU 2003280036 A1 | 19-01-2004 |
| | | CA 2491055 A1 | 08-01-2004 |
| | | CN 1665553 A | 07-09-2005 |
| | | EP 1519761 A1 | 06-04-2005 |
| | | JP 2005531365 T | 20-10-2005 |
| | | WO 2004002546 A1 | 08-01-2004 |
| US 2004166236 A1 | 26-08-2004 | AU 2002302602 B2 | 09-11-2006 |
| | | BR 0205270 A | 08-07-2003 |
| | | CA 2439075 A1 | 21-11-2002 |
| | | CN 1464981 A | 31-12-2003 |
| | | DE 10123012 C1 | 25-07-2002 |
| | | WO 02093207 A1 | 21-11-2002 |
| | | EP 1397710 A1 | 17-03-2004 |
| | | JP 2004527004 T | 02-09-2004 |
| | | MX PA03010267 A | 06-12-2004 |
| US 2008248293 A1 | 09-10-2008 | WO 2008123958 A2 | 16-10-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20020051448 A1
- US 20056943204 B2
- EP 20071860141 A1, Wang
- US 20020028330 A1
- EP 19970487418 B1
- WO 198808287 A
- US 19925091204 A
- US 5091204 A
- US 6729939 B, Wrue
- US 2003 A


### Non-patent literature cited in the description


- **Guelcher et al.** An introduction to biomaterials. Taylor Francis Group, 2003
- **Yao, K. et al.** *J Biomed. Mater. Res.,* 2006, vol. 78A, 684-692
- **Willis, S.L. et al.** *Biomaterials,* 2001, vol. 22, 3261-3272
- **Hirota, K. et al.** *FEMS Microbiol. Lett.,* 2005, vol. 248, 37-45
- **Moro, T. et al.** *Nat. Mater.,* 2004, vol. 3, 829-836
- **Guelcher et al.** An introduction to biomaterials. Taylor Francis Group, 2003
- **Dumaine.** *L. Thesis.,* 2004, 79-85
- **Oehr, C.** *Nuclear Instrum. Methods Phys. Res.,* 2003, vol. 208, 40-47
- **Evens, M.D.M. et al.** *Biomaterials,* 2001, vol. 22, 3319-3328
- **Klee, D. et al.** *Adv. Pol. Sci.,* 1999, vol. 149, 1-57
- **Denes, F.S. ; Manolache, S.** *Prog. Polym. Sci.,* 2004, vol. 29, 815-885
- **Bogaerts, A. et al.** *Spectrochemica Acta Part B,* 2002, vol. 57, 609-658
- **Chan. C.M.** *Surf Sci. Reports,* 1996, vol. 24, 1-54
- **Senesi, G.S. et al.** *Surf Sci.,* 2007, vol. 601, 1019-1025
- **Pizzoferrato, A. et al.** *Biomaterials,* 1995, vol. 16, 361-367
- **Vaswani, S.** *Academic Thesis,* 2004
- **Garrison, M.D. et al.** *Thin Solid Films,* 1999, vol. 352, 13-21
- **Hochart, F. et al.** *Appl. Surf Sci.,* 1999, vol. 42 (1-4), 574-578
- **Sterrett, T.L. et al.** *J. Mater. Sci. Mater. Med.,* 1992, vol. 3, 402-407
- **Hochart, F. et al.** *Appl. Surf Sci.,* 1999, vol. 42 (I-4), 574-578
- **Hoffman, A.S.** Annals New York Acad. Sci. 2001, vol. 944, 62-73
- **Bogaerts et al.** *Spectrochemica Acta Part B,* 2002, vol. 57, 609-658
- **Chan et al.** *Surf. Sci. Reports,* 1996, vol. 24, 1-54
- **Denes et al.** *Progr. Polym. Sci,* 2004, vol. 29, 815-885
- **Raffaelo-Addamo et al.** *Appl. Surf. Sci,* 2006, vol. 252, 2265-2275
- **Polleti et al.** *Appl. Surf. Sci.,* 2003, vol. 219, 311-316
- **Denes, F.S. et al.** *Prog. Polym. Sci.,* 2004, vol. 29, 815-885
- **Dumaine, L.** Thesis. Ecole Centrale de Lyon, 2004, 79-85
- **Bozukova, D. et al.** *Biomacromolecules,* 2007, vol. 8, 2379-2387
- **Bozukova, D.** *Langmuir,* 2008, vol. 24, 6649-6658